# EUROPEAN PATENT SPECIFICATION

(11) **EP 2 744 477 B1**
(45) Date of publication and mention of the grant of the patent: **19.09.2018**
(21) Application number: 12751685.4
(22) Date of filing: 15.08.2012
(51) Int. Cl.: A61Q 19/10, A61Q 19/00, A61K 8/27, A61K 8/49

(54) **METHODS OF ENHANCING SKIN HYDRATION AND IMPROVING NON-DISEASED SKIN**
VERFAHREN ZUR VERBESSERUNG DER HAUTHYDRIERUNG UND VERBESSERUNG VON GESUNDER HAUT
PROCÉDÉS D'AUGMENTATION DE L'HYDRATATION DE LA PEAU ET D'AMÉLIORATION DE LA PEAU NON MALADE

(30) Priority: 15.08.2011 US 201161523715 P
(43) Date of publication of application: 25.06.2014
(73) Proprietor: The Procter & Gamble Company, Cincinnati, OH 45202 (US)
(72) Inventor: STELLA, Qing, Cincinnati, Ohio 45242 (US); COOK, Jason, Edward, Anderson Township, Ohio 45230 (US)
(74) Representative: Herzog, Fiesser & Partner Patentanwälte PartG mbB
(86) International application number: PCT/US2012/050882
(87) International publication number: WO 2013/025769

(56) References cited:
- WO-A1-96/29983
- WO-A2-03/007900
- DE-A1- 19 537 509
- FR-A1- 2 685 638
- US-A1- 2011 117 225

## Description

### TECHNICAL FIELD

The present disclosure generally relates to methods of enhancing skin hydration according to claim 1.

### BACKGROUND

While non-diseased skin is generally free of major conditions like disease, infection, or fungus, people with non-diseased skin can still suffer from dryness. Accordingly, it would be desirable to provide methods for improving non-diseased skin by applying a zinc-containing and/or pyrithione material to the skin to an individual.

### SUMMARY

A method of enhancing skin hydration, the method comprising applying a rinse-off personal care composition comprising at least one of a zinc-containing material and a pyrithione material to non-diseased skin of an individual.

A method of enhancing skin hydration, the method comprising: applying a rinse-off personal care composition to non-diseased skin of an individual; the rinse-off personal composition comprising a structured surfactant, a benefit agent, and a mercaptopyridine-N-oxide zinc salt; and the non-disease skin comprising dry skin exhibiting a grade of about 2.5 or greater prior to initial application of the rinse-off personal care composition.

### BRIEF DESCRIPTION OF THE DRAWINGS

FIG. 1 shows plots of objective physical measurements of a Corneometer to determine improvement of skin hydration for three rinse-off personal care compositions, with the measurements taken 3 hours after a daily application of the rinse-off personal care compositions over a period of 21 days.
FIG. 2 shows a chart identifying measurements of a Corneometer to determine improvement of skin hydration for two rinse-off personal care compositions, with the measurements taken 24 hours and 48 hours after daily applications of the rinse-off personal care compositions after 21 treatments.
FIG. 3 illustrates that natural moisturizing factor biomarkers are enhanced deeper into the skin layer when a zinc-containing material is deposited onto the skin of an individual versus a composition without a zinc-containing material. Thus, zinc-containing materials provide an improved hydration of the deeper layers of the skin.

### DETAILED DESCRIPTION

### I. Definitions

As used herein, the following terms shall have the meaning specified thereafter:
"Anhydrous" refers to those compositions, and components thereof, which are substantially free of water.

"Biomarker" refers to any biological molecules (genes, proteins, lipids, metabolites) that can, singularly or collectively, reflect the current or predict future state of a biological system. Thus, as used herein, various biomarkers can be indicators of a quality of skin in terms of skin hydration, among several other properties. Non-limiting examples of biomarkers include inflammatory cytokines, natural moisturizing factors, one or more of keratins 1, 10 and 11, lipids and total protein. The response of skin to treatment with compositions, including personal care compositions for example, can be assessed by measuring one or more biomarkers.

"Multiphase" refers to compositions comprising at least two phases which can be chemically distinct (e.g. a cleansing phase and a benefit phase). Such phases can be in direct physical contact with one another. A personal care composition can be a multiphase personal care composition where phases of the personal care composition can be blended or mixed to a significant degree, but still be physically distinct. In these situations, the physical distinctiveness is undetectable to the naked eye. The personal care composition can be a multiphase personal care composition where the phases are in physical contact and are visually distinct. Visually distinct phases can take many forms, for example, they can appear as striped, marbled, etc.

"Natural moisturizing factor (NMF)" refers to a collection of water-soluble compounds that can be found in the stratum corneum. These compounds comprise about 20-30% of the dry weight of the corneocyte. NMF components absorb water from the atmosphere and combine the water with NMF water content, allowing the outermost layers of the stratum corneum to stay hydrated despite being exposed to external elements. Such NMFs include amino acids, lactic acid, urea, and pyrrolidone carboxylic acid (PCA).

"Non-diseased skin" refers to skin that is generally free of disease, infection, and/or fungus. As used herein, dry skin is considered to be included in non-diseased skin.

"Dry skin" is usually characterized as rough, scaly, and/or flaky skin surface, especially in low humidity conditions and is often associated with the somatory sensations of tightness, itch, and/or pain.

"Package" refers to any suitable container for a personal care composition including but not limited to a bottle, tottle, tube, jar, non-aerosol pump, and combinations thereof.

"Personal care composition" refers to compositions intended for topical application to skin and/or hair. Personal care compositions can be rinse-off formulations, in which the product can be applied topically to the skin or hair and then subsequently rinsed within seconds to minutes from the skin or hair with water. The product could also be wiped off using a substrate. In either case, it is believed at least a portion of the product is left behind (i.e. deposited) on the skin. The personal care compositions can also be used as shaving aids. The personal care compositions can be extrudable or dispensable from a package. The personal care compositions can be in the form of, for example, a liquid, semi-liquid cream, lotion, gel, or a combination thereof. Examples of personal care compositions can include but are not limited to bar soap, shampoo, conditioning shampoo, body wash, moisturizing body wash, shower gels, skin cleansers, cleansing milks, hair and body wash, in shower body moisturizer, pet shampoo, shaving preparations, and cleansing compositions used in conjunction with a disposable cleansing cloth.

"SLS" refers to sodium lauryl sulfate.

"STnS" refers to sodium trideceth(n) sulfate, wherein n can define the average number of moles of ethoxylate per molecule.

"Structured" refers to having a rheology that can confer stability on the personal care composition. A degree of structure can be determined by characteristics determined by one or more of the following methods: Young's Modulus Method, Yield Stress Method, or Zero Shear Viscosity Method or by a Ultracentrifugation Method, described in U.S. Patent No. 8,158,566, granted on April 17, 2012. A cleansing phase can be considered to be structured if the cleansing phase has one or more following characteristics: (a) Zero Shear Viscosity of at least 100 Pascal-seconds (Pa-s), at least about 200 Pa-s, at least about 500 Pa-s, at least about 1,000 Pa-s, at least about 1,500 Pa-s, or at least about 2,000 Pa-s; (b) A Structured Domain Volume Ratio as measured by the Ultracentrifugation Method, of greater than about 40%, at least about 45%, at least about 50%, at least about 55%, at least about 60%, at least about 65%, at least about 70%, at least about 75%, at least about 80%, at least about 85%, or at least about 90%; or (c) A Young's Modulus of greater than about 2 Pascals (Pa), greater than about 10 Pa, greater than about 20 Pa, greater than about 30 Pa, greater than about 40 Pa, greater than about 50 Pa, greater than about 75 Pa, or greater than about 100 Pa.

"Lather" refers to an aerated foam which results from providing energy to aqueous surfactant mixtures, particularly dilute mixtures.

The phrase "substantially free of' as used herein, unless otherwise specified means that the composition or method comprises less than about 5%, less than about 3%, less than about 1%, or even less than about 0.1% of the stated ingredient. The term "free of' as used herein means that the composition or method comprises 0% of the stated ingredient that is the ingredient has not been added to the personal care composition. However, these ingredients may incidentally form as a byproduct or a reaction product of the other components of the personal care composition.

### II. Method of Enhancing Skin Hydration and Method of Improving Non-Diseased Skin

While it is suggested in the literature that at least some zinc-containing and/or pyrithione materials have benefits on skin, for example, zinc pyrithione and its antimicrobial properties, it has been surprisingly found that zinc-containing and/or pyrithione materials can also have a newly discovered benefit of improved hydration. The improved hydration included, for example, better hydration of the deeper layers of the skin and/or longer lasting hydration. Moreover, previously reported benefits from zinc-containing and/or pyrithione materials focused on diseased skin, while it is believed the newly discovered benefit herein can also be seen on non-diseased skin.

### A. Zinc-Containing and/or Pyrithione Materials

A method of enhancing skin hydration comprises applying a zinc-containing and pyrithione material to the skin of an individual.

The zinc-containing material comprises a zinc salt of 1-hydroxy-2-pyridinethione (known as "zinc pyrithione" or "ZPT"), for example, a mercaptopyridine-N-oxide zinc salt. The ZPT can be made by reacting 1-hydroxy-2-pyridinethione (i.e., pyrithione acid) or a soluble salt thereof with a zinc salt (e.g. zinc sulfate) to form a zinc pyrithione precipitate as illustrated in U.S. Patent No. 2,809,971 and the zinc pyrithione can be formed or processed into platelet ZPT using, for example, sonic energy as illustrated in U.S. Patent No. 6,682,724.

Zinc pyrithione can take the form of particulates, platelets, or a combination thereof. For example, where the zinc pyrithione is introduced as particulate, such particulates may have an average particle size from about 0.1 µm to about 20 µm; such particulates may also have an average particle size from about 0.2 µm to about 10 µm.

Other non-limiting zinc containing materials can include zinc-containing layered materials ("ZLM's"). Examples of zinc-containing layered materials useful herein can include zinc-containing layered structures with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLM's) may have zinc incorporated in the layers and/or be components of the gallery ions. Many ZLM's occur naturally as minerals. Common examples include hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions. All of these natural materials can also be obtained synthetically or formed in situ in a composition or during a production process. Another common class of ZLM's, which are often, but not always, synthetic, is layered doubly hydroxides, which are generally represented by the formula [M²⁺_{1- x}M³⁺ₓ(OH)₂]^{x+} A^{m-}_{x/m}·nH₂O and some or all of the divalent ions (M²⁺) would be represented as zinc ions (Crepaldi, EL, Pava, PC, Tronto, J, Valim, JB J. Colloid Interfac. Sci. 2002, 248, 429-42).

Yet another class of ZLM's can be prepared and is called hydroxy double salts (Morioka, H., Tagaya, H., Karasu, M, Kadokawa, J, Chiba, K Inorg. Chem. 1999, 38, 4211-6). Hydroxy double salts can be represented by the general formula [M²⁺₁₋ₓM²⁺₁₊ₓ(OH)_{3(1-y)}]⁺ Aⁿ⁻_{(1=3y)/n}·nH₂O where the two metal ion may be different; if they are the same and represented by zinc, the formula simplifies to [Zn₁₊ₓ(OH)₂]^{2x+} 2x A⁻·nH₂O. This latter formula represents (where x=0.4) and contains common materials such as zinc hydroxychloride and zinc hydroxynitrate. These are related to hydrozincite as well wherein a divalent anion replaces the monovalent anion. These materials can also be formed in situ in a composition or in or during a production process. These classes of ZLM's represent relatively common examples of the general category and are not intended to be limiting as to the broader scope of materials which fit this definition.

Commercially available sources of basic zinc carbonate include Zinc Carbonate Basic (Cater Chemicals: Bensenville, IL, USA), Zinc Carbonate (Shepherd Chemicals: Norwood, OH, USA), Zinc Carbonate (CPS Union Corp.: New York, NY, USA), Zinc Carbonate (Elementis Pigments: Durham, UK), and Zinc Carbonate AC (Bruggemann Chemical: Newtown Square, PA, USA).

Basic zinc carbonate, which also may be referred to commercially as "Zinc Carbonate" or "Zinc Carbonate Basic" or "Zinc Hydroxy Carbonate", is a synthetic version consisting of materials similar to naturally occurring hydrozincite. The idealized stoichiometry is represented by Zn₅(OH)₆(CO₃)₂ but the actual stoichiometric ratios can vary slightly and other impurities may be incorporated in the crystal lattice.

Suitable examples of such pyrithione materials can include zinc pyrithione, sodium pyrithione, pyrithione acid, dipyrithione, chitonsan pyrithione, magnesium disulfide pyrithione, and combinations thereof. Pyrithione materials may also include other pyridinethione salts formed from heavy metals such as zinc, tin, cadmium, magnesium, aluminium, and zirconium.

### B. Applying and Depositing Zinc-Containing and/or Pyrithione Materials

To improve skin hydration and/or improve non-diseased skin, a zinc-containing and/or pyrithione material can be applied to and rinsed from the skin of an individual at least once per day for several days. Non-diseased skin treated with a zinc-containing and/or pyrithione material can show improvements in, for example, hydration level.

For example, a zinc-containing and/or pyrithione material can be applied at least once per day for about 14 days or more; or at least once per day for about 21 days or more.

The zinc-containing and/or pyrithione material can be applied directly to the skin or provided as part of a rinse-off personal care composition, which is further described herein. To achieve the enhanced hydration of the skin or improve non-diseased skin from about 0.1 µg/cm² to about 5.0 µg/cm²; from about 0.2 µg/cm² to about 5.0 µg/cm²; from about 0.5 µg/cm² to about 5.0 µg/cm²; from about 1.0 µg/cm² to about 3.0 µg/cm²; of a zinc-containing and/or pyrithione material may be deposited on the skin. Determination of the amount of zinc-containing material and/or pyrithione material deposited on the skin can be determined, for example, by using the Cup Scrub Method discussed below.

Improvements in skin hydration can be measured using known techniques, including for example, using a Corneometer, which can measure moisture level. For example, typical Corneometer Units range from about 15-20, wherein the higher the value the higher the level of hydration; and the lower the value, the lower the level of hydration. Methods for using a Corneometer are described below. The skin to which a zinc-containing and/or pyrithione material (e.g. zinc pyrithione) can be applied can exhibit a dry skin grade of about 2.5 or greater prior to a first application of the zinc-containing and/or pyrithione material. This corresponds to an average Corneometer reading of about 18 or less. The dry skin grade can be from about 2.0 to about 6.0. Once a zinc-containing and/or pyrithione material (e.g., zinc pyrithione) is applied to a desired skin surface of an individual, a measurement can be taken at predetermined time intervals to evaluate the effectiveness of the zinc-containing and/or pyrithione material for providing hydration to the skin.

For example, measurements taken 3 hours, 24 hours, or 48 hours after the zinc-containing and/or pyrithione material has been applied to the skin demonstrate that zinc-containing and/or pyrithione materials deposited on the skin can provide vast improvements to skin hydration. In fact, a Corneometer shows that about 3 hours after the 21^{st} application of the zinc-containing and/or pyrithione material to the skin, skin hydration can be improved by at least 0.5 Corneometer Units or more. Upon measuring skin hydration levels about 24 hours after the 21^{st} application of the zinc-containing and/or pyrithione material to the skin, skin hydration can be improved by at least 0.3 Corneometer Units or more. Upon measuring skin hydration levels about 48 hours after the 21^{st} application of the zinc-containing and/or pyrithione material to the skin, skin hydration can be improved by at least 0.3 Corneometer Units or more. A technique for conducting measurements using a Corneometer is described below.

Improvements in skin hydration can also be measured through the use of biomarkers. In particular, natural moisturizing factors (NMFs) can be an example of a biomarker that can be detected through methods described below. The skin which is being measured for the NMF biomarker can have a dry skin grade from about 2.5 to about 4.0 prior to the first treatment of a zinc-containing and/or pyrithione material (e.g, zinc pyrithione). One suitable method of obtaining epithelial tissue is by application of tape, such as but not limited to, any type of medical tape. This technique is well known in the art and is relatively simple to implement. The technique involves application of a tape to the epithelial tissue, typically skin, which is then removed therefrom. The biomarker analytes obtained from the epithelial tissue and present on the tape can then removed from the tape in any fashion that preserves the biomarker analytes for suitable detection and measurement assays. When at least 10 tape strips are applied, skin hydration can be improved by 0.05 units or more on a log (normalized NMF concentration) improvement index; and skin hydration can also be improved by at least 0.1 units or more on a log (normalized NMF concentration) improvement index. It is notable that where higher levels of tape strips for the biomarker testing are used, and skin hydration levels are still significant, the zinc pyrithione is deeply penetrating the skin to provide the hydration benefits. Suitable biomarkers and testing procedures for NMFs are described in U.S. Patent Application Serial No. 13/007,630.

While techniques to measure improvements in skin condition (e.g., skin hydration) can be measured using a Corneometer or biomarkers, other suitable testing methods are also available, such as methods are described in U.S. Patent Application Serial No. 13/007,630.

### III. Rinse-Off Personal Care Compositions

Zinc-containing and/or pyrithione materials (e.g., zinc pyrithione) can be applied to the skin through a rinse-off personal care composition. Suitable zinc-containing and pyrithione materials are discussed above. A rinse-off personal care composition can include a cleansing phase and a benefit phase. The benefit phase and/or cleaning phase can include the zinc-containing and/or pyrithione material (e.g. zinc pyrithione). The cleansing phase can include, for example, various surfactants as described herein. The cleaning phase and the benefit phase can be blended, and/or patterned.

The rinse-off personal care composition can comprise at least about 0.1%, by weight of the rinse-off personal care composition, of a zinc-containing and/or pyrithione material (e.g. zinc pyrithione). The rinse-off personal care composition can also comprise from about 0.2% to about 1.0%, by weight of the rinse-off personal care composition, of a zinc-containing and/or pyrithione material (e.g. zinc pyrithione). The rinse-off personal care composition can also comprise about 0.5%, by weight of the rinse-off personal care composition, of a zinc-containing and/or pyrithione material (e.g. zinc pyrithione).

### A. Cleansing Phase

As noted herein, a cleansing phase can include various surfactant chassis, and the cleansing phase can include at least one surfactant. The cleansing phase can include an aqueous structured surfactant phase. The concentration of the structured surfactant phase in the personal care composition can range from about 1% to about 20%, by weight; from about 2% to about 15%, by weight; and from about 5% to about 10%, by weight of the personal care composition.

Such a structured surfactant phase can include sodium trideceth(n) sulfate, hereinafter STnS, wherein n can define average moles of ethoxylation. n can range from about 0 to about 3. n can also range from about 0.5 to about 2.7, from about 1.1 to about 2.5, from about 1.8 to about 2.2, or n can be about 2. When n can be less than 3, STnS can provide improved stability, improved compatibility of benefit agents within the rinse-off personal care compositions, and increased mildness of the rinse-off personal care compositions, such described benefits of STnS are disclosed in U.S. Patent Application Serial No. 13/157,665.

Further, the cleansing phase can comprise a structuring system, wherein the structuring system can comprise, optionally, a non-ionic emulsifier, optionally, from about 0.05% to about 5%, by weight of the rinse-off personal care composition, of an associative polymer; and an electrolyte. The rinse-off personal care composition can be optionally free of sodium lauryl sulfate, hereinafter SLS, and can comprise at least a 70% lamellar structure. The cleansing phase can comprise at least one surfactant, wherein the at least one surfactant includes SLS. Suitable examples of SLS are described in U.S. Patent Application Serial No. 12/817,786. The at least one surfactant can include sodium laureth(n) sulfate, hereinafter SLEnS, wherein n can define average moles of ethoxylation. n can range from about 1 to about 3.

The rinse-off personal care composition can further comprise from about 0.1% to 20%, by weight of the rinse-off personal care composition, of a cosurfactant. Cosurfactants can comprise amphoteric surfactants, zwitterionic surfactants, or mixtures thereof. The rinse-off personal care composition can include at least one of an amphoteric surfactant and a zwitterionic surfactant. Suitable amphoteric or zwitterionic surfactants can include those described in U.S. Patent No. 5,104,646 and U.S. Patent No. 5,106,609.

Amphoteric surfactants can include those that can be broadly described as derivatives of aliphatic secondary and tertiary amines in which an aliphatic radical can be straight or branched chain and wherein an aliphatic substituent can contain from about 8 to about 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,378. Other examples of amphoteric surfactants can include sodium lauroamphoacetate, sodium cocoamphoactetate, disodium lauroamphoacetate disodium cocodiamphoacetate, and mixtures thereof. Amphoacetates and diamphoacetates can also be used.

Zwitterionic surfactants suitable for use can include those that are broadly described as derivatives of aliphatic quaternary ammonium, phosphonium, and sulfonium compounds, in which aliphatic radicals can be straight or branched chains, and wherein an aliphatic substituent can contain from about 8 to about 18 carbon atoms such that one carbon atom can contain an anionic group, e.g., carboxy, sulfonate, sulfate, phosphate, or phosphonate. Other zwitterionic surfactants can include betaines, including cocoamidopropyl betaine.

Other suitable surfactants or cosurfactants that can generally be used in a cleansing phase for a rinse-off personal care composition are described in McCutcheon's: Detergents and Emulsifiers North American Edition (Allured Publishing Corporation 1947) (1986), McCutcheon's, Functional Materials North American Edition (Allured Publishing Corporation 1973) (1992) and U.S. Patent No. 3,929,678 (filed Aug. 1, 1974).

A cleansing phase of a rinse-off personal care composition can also include an associative polymer. Such associative polymer can be a crosslinked, alkali swellable, associative polymer comprising acidic monomers and associative monomers with hydrophobic end groups, whereby the associative polymer comprises a percentage hydrophobic modification and a hydrophobic side chain comprising alkyl functional groups. Without intending to be limited by theory, it is believed the acidic monomers can contribute to an ability of the associative polymer to swell in water upon neutralization of acidic groups; and associative monomers anchor the associative polymer into structured surfactant hydrophobic domains, e.g., lamellae, to confer structure to the surfactant phase and keep the associative polymer from collapsing and losing effectiveness in a presence of an electrolyte. The crosslinked, associative polymer can comprise a percentage hydrophobic modification, which is a mole percentage of monomers expressed as a percentage of a total number of all monomers in a polymer backbone, including both acidic and other non-acidic monomers. Percentage hydrophobic modification of the associative polymer, hereafter %HM, can be determined by the ratio of monomers added during synthesis, or by analytical techniques such as proton nuclear magnetic resonance (NMR). Associative alkyl side chains can comprise, for example, butyl, propyl, stearyl, steareth, cetyl, lauryl, laureth, octyl, behenyl, beheneth, steareth, or other linear, branched, saturated, or unsaturated alkyl or alketh hydrocarbon side chains. The acidic monomer can comprise any acid functional group, for example sulfate, sulfonate, carboxylate, phosphonate, or phosphate or mixtures of acid groups. The acidic monomer can comprise a carboxylate, alternatively the acidic monomer is an acrylate, including acrylic acid and/or methacrylic acid. The acidic monomer comprises a polymerizable structure, e.g., vinyl functionality. Mixtures of acidic monomers, for example acrylic acid and methacrylic acid monomer mixtures, are useful.

The associative monomer can comprise a hydrophobic end group and a polymerizable component, e.g., vinyl, which can be attached. The hydrophobic end group can be attached to the polymerizable component, hence to the polymer chain, by different means but can be attached by an ether or ester or amide functionality, such as an alkyl acrylate or a vinyl alkanoate monomer. The hydrophobic end group can also be separated from the chain, for example, by an alkoxy ligand such as an alkyl etherThe associative monomer can be an alkyl ester, an alkyl (meth)acrylate, where (meth)acrylate is understood to mean either methyl acrylate or acrylate, or mixtures of the two.

The hydrophobic end group of the associative polymer can be incompatible with the aqueous phase of the composition and can associate with lathering surfactant hydrophobe components. Without intending to be limited by theory, it is believed that longer alkyl chains of structuring polymer hydrophobe end groups can increase incompatibility with the aqueous phase to enhance structure, whereas somewhat shorter alkyl chains having carbon numbers closely resembling lathering surfactant hydrophobes (e.g., 12 to 14 carbons) or multiples thereof (for bilayers, e.g.) can also be effective. An ideal range of hydrophobic end group carbon numbers combined with an optimal percentage of hydrophobic monomers expressed as a percentage of the polymer backbone can provide increased structure to the lathering, structured surfactant composition at low levels of polymer structurant.

The associative polymer can be Aqupec SER-300 made by Sumitomo Seika of Japan, which is Acrylates/C10-30 alkyl acrylate crosspolymer and comprises stearyl side chains with less than about 1% HM. Other associative polymers can comprise stearyl, octyl, decyl and lauryl side chains. Associative polymers can be Aqupec SER-150 (acrylates/C10-30 alkyl acrylates crosspolymer) comprising about C18 (stearyl) side chains and about 0.4% HM, and Aqupec HV-701EDR which comprises about C8 (octyl) side chains and about 3.5% HM. The associative polymer can be Stabylen 30 manufactured by 3V Sigma S.p.A., which has branched isodecanoate hydrophobic associative side chains.

Other optional additives can be included in the cleaning phase, including for example an emulsifier (e.g., non-ionic emulsifier) and electrolyes. Suitable emulsifiers and electrolytes are described in U.S. Patent Application Serial No. 13/157,665.

### B. Benefit Phase

As noted herein, rinse-off personal care compositions include a benefit phase. The benefit phase can be hydrophobic and/or anhydrous. The benefit phase can also be substantially free of surfactant.

The benefit phase includes a benefit agent. In particular, the benefit phase can comprise from about 0.1% to about 50%, by weight of the rinse-off personal care composition, of the benefit agent. The benefit phase can also include from about 0.5% to about 20%, by weight of the rinse-off personal care composition, of the benefit agent. Examples of the benefit agent include castor oil, petrolatum, glyceryl monooleate, mineral oil, soybean oil, and mixtures thereof.

The benefit phase can typically comprise one or more benefit agents, as set forth above. Additional examples of benefit agents can include water insoluble or hydrophobic benefit agents. As set forth above, the benefit phase can comprise from about 0.1% to about 50%, by weight of the rinse-off personal care composition, of the benefit agent.

Non-limiting examples of glycerides suitable for use as hydrophobic skin benefit agents herein can include castor oil, safflower oil, corn oil, walnut oil, peanut oil, olive oil, cod liver oil, almond oil, avocado oil, palm oil, sesame oil, soybean oil, vegetable oils, sunflower seed oil, vegetable oil derivatives, coconut oil and derivatized coconut oil, cottonseed oil and derivatized cottonseed oil, jojoba oil, cocoa butter, petrolatum, mineral oil, and combinations thereof.

Non-limiting examples of alkyl esters suitable for use as hydrophobic skin benefit agents herein can include isopropyl esters of fatty acids and long chain esters of long chain (i.e. C10-C24) fatty acids, e.g., cetyl ricinoleate, non-limiting examples of which can include isopropyl palmitate, isopropyl myristate, cetyl riconoleate, and stearyl riconoleate. Other example can include hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use as hydrophobic skin benefit agents herein can include oleyl myristate, oleyl stearate, oleyl oleate, and combinations thereof.

Non-limiting examples of polyglycerin fatty acid esters suitable for use as hydrophobic skin benefit agents herein can include decaglyceryl distearate, decaglyceryl diisostearate, decaglyceryl monomyriate, decaglyceryl monolaurate, hexaglyceryl monooleate, and combinations thereof.

Non-limiting examples of lanolin and lanolin derivatives suitable for use as hydrophobic skin benefit agents herein can include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate, and combinations thereof.

Non-limiting examples of silicone oils suitable for use as hydrophobic skin benefit agents herein can include dimethicone copolyol, dimethylpolysiloxane, diethylpolysiloxane, mixed C1-C30 alkyl polysiloxanes, phenyl dimethicone, dimethiconol, and combinations thereof. Nonlimiting examples of silicone oils useful herein are described in U.S. Patent No. 5,011,681. Still other suitable hydrophobic skin benefit agents can include milk triglycerides (e.g., hydroxylated milk glyceride) and polyol fatty acid polyesters.

Additional optional ingredients can also be added to the rinse-off personal care composition for treatment of the skin, or to modify the aesthetics of the rinse-off personal care composition as is the case with perfumes, colorants, dyes or the like. Optional materials useful in products herein can be categorized or described by their cosmetic and/or therapeutic benefit or their postulated mode of action or function. However, it can be understood that actives and other materials useful herein can, in some instances, provide more than one cosmetic and/or therapeutic benefit or function or operate via more than one mode of action. Therefore, classifications herein can be made for convenience and cannot be intended to limit an ingredient to particularly stated application or applications listed. A precise nature of these optional materials, and levels of incorporation thereof, will depend on the physical form of the composition and the nature of the cleansing operation for which it is to be used. Optional materials can usually be formulated at about 6% or less, about 5% or less, about 4% or less, about 3% or less, about 2% or less, about 1% or less, about 0.5% or less, about 0.25% or less, about 0.1% or less, about 0.01% or less, or about 0.005% or less of the rinse-off personal care composition.

To further improve stability under stressful conditions such as high temperature and vibration, densities of separate phases can be adjusted such that they can be substantially equal. To achieve this, low density microspheres can be added to one or more phases of the rinse-off personal care composition. Examples of rinse-off personal care compositions that comprise low density microspheres are described in a patent application published on May 13, 2004 under U.S. Patent Publication No. 2004/0092415A1 entitled "Striped Liquid Personal Cleansing Compositions Containing A Cleansing Phase and A Separate Phase with Improved Stability," filed on Oct. 31, 2003 by Focht, et al.

Other non-limiting optional ingredients that can be used in the personal care composition can comprise an optional benefit component that can be selected from the group consisting of thickening agents; preservatives; antimicrobials; fragrances; chelators (e.g. such as those described in U.S. Patent No. 5,487,884 issued to Bisset, et al.); sequestrants; vitamins (e.g. Retinol); vitamin derivatives (e.g. tocophenyl actetate, niacinamide, panthenol); sunscreens; desquamation actives (e.g. such as those described in U.S. Patent No. 5,681,852 and 5,652,228 issued to Bisset); anti-wrinkle/ anti-atrophy actives (e.g. N-acetyl derivatives, thiols, hydroxyl acids, phenol); anti-oxidants (e.g. ascorbic acid derivatives, tocophenol) skin soothing agents/skin healing agents (e.g. panthenoic acid derivatives, aloe vera, allantoin); skin lightening agents (e.g. kojic acid, arbutin, ascorbic acid derivatives) skin tanning agents (e.g. dihydroxyacteone); anti-acne medicaments; essential oils; sensates; pigments; colorants; pearlescent agents; interference pigments (e.g such as those disclosed in U.S. Patent No. 6,395,691 issued to Liang Sheng Tsaur, U.S. Patent No. 6,645,511 issued to Aronson, et al., U.S. Patent No. 6,759,376 issued to Zhang, et al, U.S. Patent No. 6,780,826 issued to Zhang, et al.) particles (e.g. talc, kolin, mica, smectite clay, cellulose powder, polysiloxane, silicas, carbonates, titanium dioxide, polyethylene beads) hydrophobically modified non-platelet particles (e.g. hydrophobically modified titanium dioxide and other materials described in a commonly owned, patent application published on Aug. 17, 2006 under Publication No. 2006/0182699A, entitled "Personal Care Compositions Containing Hydrophobically Modified Non-platelet particle filed on Feb. 15, 2005 by Taylor, et al.) and mixtures thereof. The multiphase personal care composition can comprise from about 0.1% to about 4%, by weight of the rinse-off personal care composition, of hydrophobically modified titanium dioxide. Other such suitable examples of such skin actives are described in U.S. Patent Application Serial No. 13/157,665.

Other optional ingredients can be most typically those materials approved for use in cosmetics and that are described in the CTFA Cosmetic Ingredient Handbook, Second Edition, The Cosmetic, Toiletries, and Fragrance Association, Inc. 1988, 1992.

### IV. Procedures

### A. Cup Scrub Procedure

As noted herein, the Cup Scrub Procedure can be used to assist in determining how much zinc-containing and/or pyrithione material is deposited onto the skin of an individual. First, test subjects wet the volar forearm surface under running water (flow = 4.5 L/min, temp = 35-38°C) for approximately 15 seconds. Next, the test subjects dose 1 mL of body wash (via disposable syringe) to the volar forearm surface. The subjects proceed to generate lather on the volar forearm by rubbing the applied body wash with their opposite hand for approximately 15 seconds. Following the 15-second lathering process, the lather is allowed to sit undisturbed on the skin for an additional 15 seconds. At the end of the 15-second wait (30 seconds after the start of the lathering process), the subjects rinse the arm for approximately 10 seconds, allowing the running water to contact the proximal volar forearm surface and cascade down (toward the distal surface). Following the rinse, the subjects use a paper towel to pat the surface dry.

The next part of the procedure involves a 2-cm diameter glass cylinder containing a bead of silicone caulking on a skin contact edge which will be pressed firmly against a skin surface to prevent leakage of an extraction fluid. One mL of the extraction solvent can be pipetted into the glass cylinder. To determine how much zinc pyrithione is deposited, for example, the extraction solvent can be 80:20 0.05 M EDTA:EtOH. While using a transfer pipette or glass rod, an entire area within the glass cylinder can be scrubbed for about 30 seconds using moderate pressure. The solution can be removed and pipetted into a labeled glass sample vial. The Cup Scrub Procedure can be repeated using fresh extraction solution, which will be pooled with the initial extraction in the labeled vial.

After each use, the glass cylinder and rod can be cleaned. For example, each cylinder and rod can be immersed in dilute Dawn® solution and scrubbed with a finger or soft bristle brush. The cylinders and rods can then be immersed in IPA. Finally, cylinders and rods can be wiped dry with a Kimwipe or other lint free tissue to remove any visible residue. Scrub solutions can be changed at an end of each day or when any visible layer of residue can be found in the bottom thereof. Further, samples can be stored at 4°C (± 3°C) until the samples can be submitted for HPLC analysis. HPLC analysis is then used to determine the amount of deposition. The free pyrithione in solution is then derivatized with 2-2'-Dithiopyridine, and subsequently analyzed via HPLC utilizing UV detection. The results are reported as µg of zinc pyrithione per mL of solution.

### B. Dry Skin Grade Screen and Application of Materials for Corneometer and NMF Testing

Test subjects are screened for dry skin grade of 2.5-4.0 by trained expert graders following guidelines below. Visual evaluations will be done with the aid of an Illuminated Magnifying Lamp which provides 2.75X magnification and which has a shadow-free circular fluorescent light source (General Electric Cool White, 22 watt 8" Circline). At least 30 subjects are needed to obtain sufficient replicates for each treatment. Table 1 shows a grading scale for dry skin and lists the redness and dryness characteristics associated with each grade.

Table 1.

| Grade* | Redness | Dryness** |
|---|---|---|
| 0.0 | No redness | Perfect skin |
| 1.0 | Barely detectable redness | Patches of checking and/or slight powderiness, occasional patches of small scales may be seen, distribution generalized |
| 2.0 | Slight redness | Generalized slight powderiness, early cracking, or occasional small lifting scales may be present |
| 3.0 | Moderate redness | Generalized moderate powderiness and/or heavy cracking and lifting scales |
| 4.0 | Heavy or substantial redness | Generalized heavy powderines s and/or heavy cracking and lifting scales |
| 5.0 | Severe redness | Generalized high cracking and lifting scales, eczematous change may be present, but not prominent, may see bleeding cracks |
| 6.0 | Extreme redness | Generalized severe cracking, bleeding cracks and eczematous changes may be present, large scales may be sloughing off |
| *Half-unit grades may be used if necessary | | |
| **"Generalized" refers to situations where more than 50% of an application area is affected | | |

Before initial visual grading, a clinical assistant will mark 2-7 cm (across) x 10 cm (down) treatment sites on an outer portion of the lower legs using a template and a laboratory marking pen (4 corner brackets are sufficient to delineate each area). For assignment of the products, two sites located on the left leg will be numbered L1 and L2, where L1 is the top part of the lower leg nearest the knee, and L2 is the bottom part of the lower leg nearest the ankle. Two sites located on the right leg will be numbered R1 and R2, where R1 is the top part of the lower leg nearest the knee, and R2 is the bottom part of the lower leg nearest the ankle.

To simplify the treatment process, master trays will be prepared for each treatment plan specified in the study randomization. Each master tray will be divided in half, with each half labeled 'left' or 'right' to indicate which leg it corresponds to, then subdivided into sections for the test products in the order of leg application site. One or more make-up trays can also be prepared for use as needed using individual coded containers, or other appropriate product code indicators, that can be re-arranged according to a given treatment plan.

Trained clinical assistants will wash each subject's lower legs in a controlled manner with assigned treatments once daily for 21 consecutive days. Assignment of test treatments to skin sites on the left and right legs will be designated by study randomization. A target dose of body wash for each site is 10 µL/cm². All body wash products will be dispensed at 0.7 mL dosages. All body wash test products will be drawn up into syringes at the 0.7 mL dosage. A one day supply of syringes for all products may be filled the day before or the day of use. Product that has been transferred to another container and the container itself will be used for one day only (i.e., the day the transfer occurred). All syringe filling operations will be appropriately documented (e.g., product code filled, when filled, initials of person responsible for filling).

The treatment area on the top part of the left leg of the subject is wetted for 5 seconds with 95-100°F running tap water. The water flow rate is about 1200 mL per minute. For the "No Treatment" site, apply water only. For a treatment site, dispense 0.7 mL of body wash product from the syringe onto the center of the treatment area and place a wet puff over the dispensed product and gently rub the puff back and forth within the treatment site for 10 seconds. Then, allow lather (or water only) to remain on the site for 90 seconds. When residence time for a site has expired, the site is rinsed for 15 seconds under a running tap, taking care not to rinse adjacent sites. After the application area has been rinsed, the area is gently patted dry. Repeat the procedure for the lower part of the left leg, and after completion, use the same procedure for each of the top part of the right leg and the lower part of the right leg.

### C. Corneometer Testing

Once the materials are applied as noted above in Section B, improvements in skin hydration can be measured with a Corneometer, while baseline measurements are taken prior to application of materials. In particular, skin hydration based upon measurements of capacitance can be assessed using the Corneometer® 825 as set forth in U.S. Patent Application Serial No. 13/007,630. Such measurements can be non-invasive and can be taken in duplicate on each site of the subjects' legs at the following times: At baseline, prior to 1^{st} treatment; 3 hours post 1^{st}, 3^{rd}, 5^{th} 14^{th} and 21^{st} treatments; 24 hours post 4^{th}, 13^{th} and 21^{st}, treatments, 48 hours post 21^{st} treatment after a visual assessment has been completed. Subjects can be acclimated for a minimum of thirty minutes in an environmentally controlled room (maintained at 70°F ± 2 and 30-45% relative humidity) prior to the non-invasive instrumental measurements taken on their legs. Data can be recorded electronically using a Sponsor's direct data entry and data capture programs. Measurements can be performed according to a test facility's standard operating procedures and/or the Sponsors Instrument Operation Manual.

The Corneometer values are arbitrary units for electrical impedance. At baseline, for subjects having a dry skin grade from about 2.5 to about 4.0, an adjusted mean of such Corneometer values can typically fall within a range of about 15 to about 20. Higher Corneometer values can correspond to a higher hydration level, and thus, lower Corneometer values can correspond to lower hydration levels.

The instrument should only be operated by trained operators. Further, the same instrument(s) and operator(s) can be used throughout the study. Kimwipes can be used to wipe an end of a probe. The probe can be wiped with a Kimwipe between each measurement. At the end of an evaluation session, data collected for that period can be backed up according to instructions in the Sponsors Instrument Operation Manual, and a hard copy of the data can be printed.

### D. Biomarkers: Natural Moisturizing Factors (NMFs)

Biomarkers that can be indicative of skin health can be measured to evaluate changes on one or more surfaces of epithelial tissue of a subject caused by a test product. Thus, biomarkers can allow for a relatively simple, efficient and quick determination of the usefulness of a test product for providing one or more benefits to skin.

Samples of epithelial tissue can be obtained to collect and analyze biomarker analytes. Non-limiting examples of suitable obtaining techniques can include application of tape, rinsing by lavage method, biopsy, swabbing, scraping, blotting and combinations thereof. However, whichever obtaining technique is used, it should be one where the biomarkers obtained are those present on the surface and/or in the epithelial tissue, and not those included on any of the underlying non-epithelial tissue, such as muscle.

A method of obtaining epithelial tissue can be by application of tape, such as but not limited to, any type of medical tape. A technique of applying tape can involve application of a tape to the skin and then removal therefrom. Biomarker analytes obtained from the skin and present on the tape can be removed from the tape in any fashion such that the biomarker analytes can be preserved for suitable detection and measurement assays. Examples of tapes can include, but are not limited to: D-squame tape®, and SEBUTAPE®, both of which are available from CuDerm Corporation, Dallas, Tex., USA; and Transpore® tape which is available from the 3M company, of Minnesota USA.

Biomarker analytes can be present in test and control samples and can be identified using one or more techniques known in the art. Detection techniques such as antibodies, nucleotide probes, highly specific chemical tags, markers, dyes, enzyme linked and other colorimetric and fluorometric probes and assays can be used to detect and measure biomarker analytes. In some non-limiting examples, biomarker analytes can include inflammatory cytokines, natural moisturizing factors (NMFs), keratin 1, keratin 10, keratin 11, lipids and total protein.

Examples of NMFs can include amino acids, lactic acid, urea, and pyrrolidone carboxylic acid (PCA), and more particularly include Trans-Urocanic Acid, Citrulline, Glycine, Histidine, Ornithine, Proline, 2 Pyrrolidone 5 Acid, and Serine. As set forth above, effectiveness of treatment with a test composition can evidenced by an increase in the amount of NMFs. NMFs can be measured to detect improvement in skin hydration. Such methodology is futher described in U.S. Patent Application No. 13/007,630.

To measure NMF values, tape strips (D-Squame) from subjects are placed into polypropylene tubes and vortexed or sonicated with acidified water to extract relevant amino acid related NMFs (glycine, histidine, proline, serine, urocanic acid, citrulline ornithine and 2-Pyrrolidone5-carboxylic acid). Extracts from the tape strips are spiked with stable-isotope internal standards of each NMF and then analyzed by gradient reversed-phase high performance liquid chromatography with tandem mass spectrometry using multiple-reaction-monitoring. Combined standards for the NMFs are prepared over the required concentration range, spiked with the stable-isotope internal standards, and analyzed along with the samples. The response ratio of each standard (response of standard/response of internal standard) for each NMF are plotted versus the standard concentration to generate a regression curve for each of the NMFs. The concentration of each NMF in the extracts is then determined by interpolation from the appropriate regression standard curve.

### V. Examples

### A. Example 1

Table 2 illustrates three formulations for rinse-off personal care compositions, which shows the amount of the component added in the form in which it is received from a supplier. Thus, if a component is received as a solution, the amount of the added solution is contained below. Composition A and Composition B are comparative examples for Composition C, which contains 0.5% zinc pyrithione. For each test described below, subjects having a dry skin grade of from about 2.5 to about 4.0 were tested.

**Table 2.**

| Ingredient | Composition A, wt. % | Composition B, wt. % | Composition C, wt. % |
|---|---|---|---|
| Distilled Water | Q.S. | Q.S. | Q.S. |
| Sodium Tridecyl Ether Sulfate | 10.54 | 10.54 | 10.54 |
| Dehyton ML | 6.59 | 6.59 | 6.59 |
| Electrolyte | 4.01 | 4.01 | 4.01 |
| Iconol TDA3-Ethoxylated Tridecyl Alcohol | 0.84 | 0.84 | 0.84 |
| Cationic Polymer | 0.35 | 0.35 | 0.35 |
| SodiumBenzoate, NF | 0.24 | 0.24 | 0.24 |
| pH Adjustment Agent | 0.23 | 0.23 | 0.23 |
| Aqupec Ser W-300C | 0.17 | 0.17 | 0.17 |
| Dissovine na2-s | 0.13 | 0.13 | 0.13 |
| Kathon CG | 0.031 | 0.031 | 0.031 |
| Hydrogen peroxide solution, 20-40% | 0.004 | 0.004 | 0.004 |
| Soybean Oil | 15 | -- | -- |
| Petrolatume | -- | 13 | 12.5 |
| Glyceryl monooleate | -- | 2 | 2 |
| Mercaptopyridine-N-oxide (ZPT) | -- | -- | 0.5 |

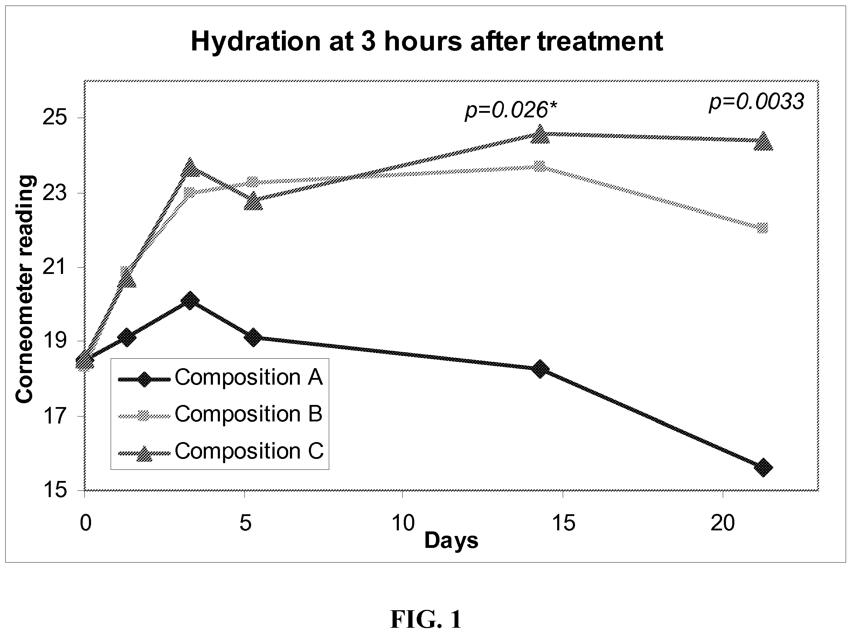

FIG. 1 shows plots of Corneometer measurements taken over the course of 21 days, wherein each measurement was taken 3 hours after application to the skin. Measurements taken at each of the 14^{th} day and the 21^{st} day indicate that Composition C, containing zinc pyrithione, provides a higher Corneometer reading than the control compositions, Composition A and Composition B, and thus, provides a greater improvement in skin hydration.

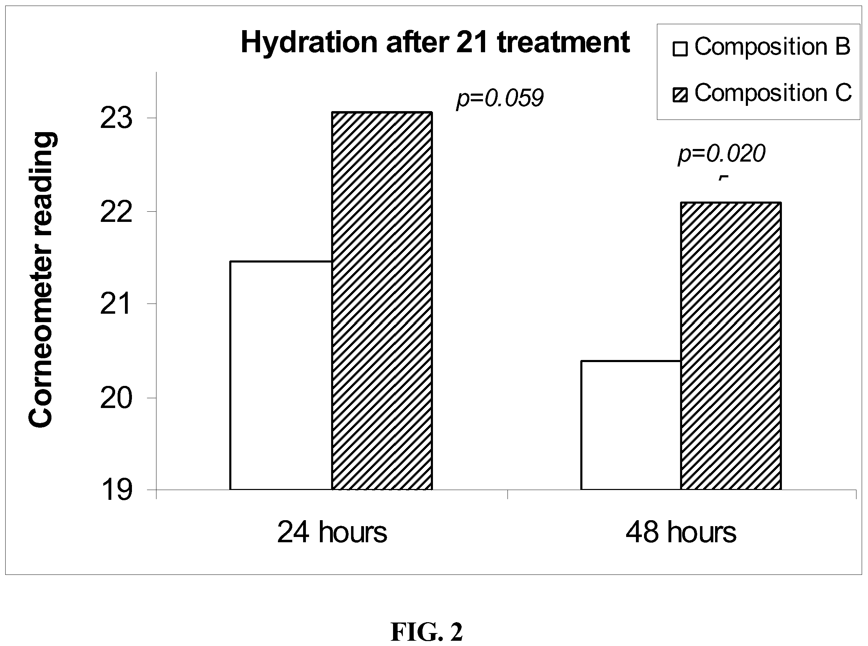

FIG. 2 shows a chart that includes measurements at 24 hours and 48 hours after 21 treatments for each of Composition B and Composition C. As with results from FIG. 1, FIG.2 shows that Composition C, containing zinc pyrithione, provides a greater improvement in skin hydration.

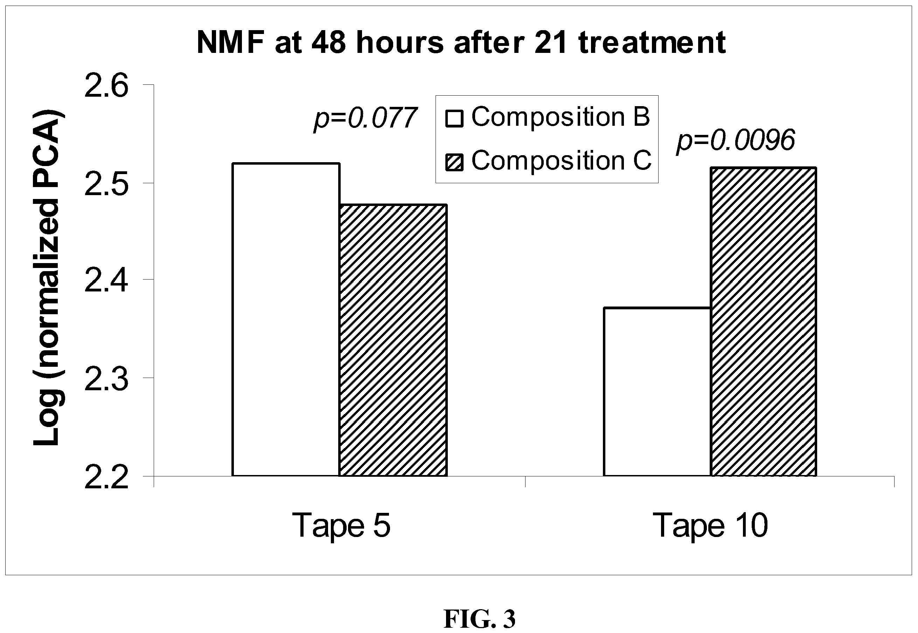

FIG. 3 shows a biomarker analysis of the depth effect of NMF (NMF used here is PCA) and the benefits achieved by depositing ZPT onto the skin of an individual. Measurements were taken after use of 5 tape strips and 10 tape strips for each of Composition B and Composition C. As indicated, Composition C illustrated much higher hydration levels at the 10 tape strips measurement, indicating the enhanced hydration benefits in deeper layers of stratum corneum provided by the addition of ZPT.

### B. Example 2

Table 3 shows a representative formulation (Composition D) of a rinse-off personal care composition having SLS as a surfactant chassis.

**Table 3.**

| Ingredient | Composition D, wt.% |
|---|---|
| Distilled Water | Q.S. |
| Sodium Laureth-1-Sulfate | 6.07 |
| Laury lamido propyl Betaine (Amphosol LB) | 2.43 |
| Sodium Benzoate | 0.25 |
| EDTA (Dis s olvine Na2S) | 0.1 |
| pH Adjustment Agent | 0.1 |
| Kathon CG | 0.0003 |
| Electrolyte | 1.25 |
| Castor Oil | 1 |
| AM:Triquat (95:5) (Polyquatemium-76) | 0.3 |
| Mercaptopyridine-N-oxide (ZPT) | 0.5 |

### C. Example 3

Table 4 shows two representative formulations (Composition E and Composition F) of a rinse-off personal care composition. The compositions illustrated in the following Examples are prepared by conventional formulation and mixing methods, an example of which is described above. All exemplified amounts exclude minor materials such as diluents, preservatives, color solutions, imagery ingredients, botanicals, and so forth, unless otherwise specified.

**Table 4.**

| Ingredient | Composition E, wt.% | Composition F, wt.% |
|---|---|---|
| Water | Q.S. | Q.S |
| Guar Hydroxy Propyl Trimonium Chloride | 0.2 | - |
| AM:TRIQUAT Copolymer | 0.2 | 0.2 |
| Sodium Laureth Sulfate, n=1 | 10.5 | 6 |
| Sodium Lauryl Sulfate | - | 7 |
| Cocoamdopropyl Betaine | 1 | 1 |
| Ethylene Glycol Disterate | 2 | 2 |
| 330M silicone | 1.1 | - |
| Aminosilicone | - | 1.4 |
| Sodium Chloride | Up to 1.5% | Up to 1.5% |
| Fragrance | 0.75 | 0.75 |
| Preservatives, pH adjusters | Up to 1.3% | Up to 1.3% |
| Zinc Pyrithione | 1 | 1 |
| Zinc Hydroxy Carbonate | 1.61 | 1.61 |
| Petrolatum | 1 | 1 |
| Sodium Xylenes u lfon ate | Up to 1% | Up to 1% |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of enhancing skin hydration, the method comprising applying a rinse-off personal care composition comprising
a) a cleaning phase; and
b) a benefit phase, wherein the benefit phase comprises a benefit agent wherein the benefit agent is selected from the group consisting of petrolatum, glyceryl monooleate, mineral oil, castor oil, soybean oil, and mixtures thereof, and a zinc containing material comprising zinc pyrithione,
to non-diseased skin of an individual, wherein the non-diseased skin comprises dry skin.

2. The method of claim 1, wherein the cleansing phase comprises:
a) an aqueous structured surfactant phase comprising from 5 % to 20 %, by weight of the rinse-off personal care composition, of sodium trideceth(n) sulfate (STnS) where n is from 0.5 to 2.7;
b) at least one of an amphoteric surfactant and a zwitterionic surfactant; and
c) a structuring system comprising an electrolyte.

3. The method of claim 2, wherein the structuring system further comprises a non-ionic emulsifier and from 0.05 % to 5 %, by weight of the rinse-off personal care composition, of an associative polymer.

4. The method of any preceding claim, wherein the rinse-off personal care composition is free of sodium lauryl sulfate and comprises at least a 70 % lamellar structure.

5. The method of claims 1-4, wherein the cleansing phase comprises at least one surfactant, wherein the at least one surfactant comprises sodium laureth(n) sulfate (SLEnS) where n is between 1 and 3.

6. The method of any preceding claim, wherein the zinc containing material comprising zinc pyrithione is applied at least once per day for 14 days or more.

7. The method of preceding claim, wherein the zinc containing material comprising zinc pyrithione is applied at least once per day for 21 days or more.

8. The method of claims 1-5, wherein 0.5 µmg/cm² or more of zinc pyrithione is deposited on to the non-diseased skin during application of the personal care product.

9. The method according to any preceding claim, wherein skin hydration improves by 0.5 Corneometer Units or more 3 hours after applying the rinse-off personal care composition.

10. The method according to any preceding claim, wherein the dry skin exhibits a grade of 2.5 or greater prior to an initial application of the rinse-off personal care composition.

## Patentansprüche

1. Verfahren zum Verbessern der Hauthydratation, wobei das Verfahren das Auftragen einer Körperpflegezusammensetzung zum Abspülen umfasst, umfassend
a) eine Reinigungsphase; und
b) eine Wirkphase, wobei die Wirkphase einen Wirkstoff umfasst, wobei der Wirkstoff ausgewählt ist aus der Gruppe bestehend aus Petrolatum, Glycerylmonooleat, Mineralöl, Rizinusöl, Sojaöl und Mischungen davon und einem zinkhaltigen Material, umfassend Zink-Pyrithion,
auf nicht erkrankte Haut eines Individuums, wobei die nicht erkrankte Haut trockene Haut umfasst.

2. Verfahren nach Anspruch 1, wobei die Reinigungsphase umfasst:
a) eine wässrige, strukturierte Tensidphase, umfassend von 5 bis 20 Gew.-% der Körperpflegezusammensetzung zum Abspülen Natriumtride-ceth(n)sulfat (STnS), wobei n von 0,5 bis 2,7 beträgt;
b) mindestens eines von einem amphoteren Tensid und einem zwitterionischen Tensid; und
c) ein Strukturierungssystem, umfassend einen Elektrolyten.

3. Verfahren nach Anspruch 2, wobei das Strukturierungssystem ferner einen nichtionischen Emulgator und von 0,05 bis 5 Gew.-% der Körperpflegezusammensetzung zum Abspülen ein assoziatives Polymer umfasst.

4. Verfahren nach einem der vorstehenden Ansprüche, wobei die Körperpflegezusammensetzung zum Abspülen frei von Natriumlaurylsulfat ist und mindestens eine 70 %ige lamellare Struktur umfasst.

5. Verfahren nach den Ansprüchen 1 bis 4, wobei die Reinigungsphase mindestens ein Tensid umfasst, wobei das mindestens eine Tensid Natriumlaureth(n)sulfat (SLEnS) umfasst, wobei n zwischen 1 und 3 beträgt.

6. Verfahren nach einem der vorstehenden Ansprüche, wobei das Zink-Pyrithion umfassende zinkhaltige Material mindestens einmal pro Tag für 14 Tage oder mehr aufgetragen wird.

7. Verfahren nach dem vorstehenden Anspruch, wobei das Zink-Pyrithion umfassende zinkhaltige Material mindestens einmal pro Tag für 21 Tage oder mehr aufgetragen wird.

8. Verfahren nach den Ansprüchen 1 bis 5, wobei 0,5 µmg/cm² oder mehr Zink-Pyrithion auf die nicht erkrankte Haut während der Anwendung des Körperpflegeprodukts abgeschieden wird.

9. Verfahren nach einem der vorstehenden Ansprüche, wobei sich die Hauthydratation 3 Stunden nach dem Aufbringen der Körperpflegezusammensetzung zum Abspülen um 0,5 Corneometereinheiten oder mehr verbessert.

10. Verfahren nach einem der vorstehenden Ansprüche, wobei die trockene Haut einen Grad von 2,5 oder größer vor einer anfänglichen Anwendung der Körperpflegezusammensetzung zum Abspülen aufweist.

## Revendications

1. Procédé d'amélioration de l'hydratation de la peau, le procédé comprenant l'application d'une composition de soins personnels à éliminer par rinçage comprenant
a) une phase de nettoyage ; et
b) une phase bénéfique, dans lequel la phase bénéfique comprend un agent bénéfique dans lequel l'agent bénéfique est choisi dans le groupe constitué de vaseline, mono-oléate de glycéryle, huile minérale, huile de ricin, huile de soja, et leurs mélanges, et un matériau contenant du zinc comprenant de la pyrithione de zinc,
sur une peau non malade d'un sujet, dans lequel la peau non malade comprend une peau sèche.

2. Procédé selon la revendication 1, dans lequel la phase nettoyante comprend :
a) une phase agent tensioactif structurée aqueuse comprenant de 5 % à 20 %, en poids de la composition de soins personnels à éliminer par rinçage, de tridéceth(n)-sulfate de sodium (STnS) où n va de 0,5 à 2,7 ;
b) au moins l'un parmi un agent tensioactif amphotère et un agent tensioactif zwittérionique ; et
c) un système structurant comprenant un électrolyte.

3. Procédé selon la revendication 2, dans lequel le système structurant comprend en outre un émulsifiant non ionique et de 0,05 % à 5 %, en poids de la composition de soins personnels à éliminer par rinçage, d'un polymère associatif.

4. Procédé selon l'une quelconque des revendications précédentes, dans lequel la composition de soins personnels à éliminer par rinçage est exempte de laurylsulfate de sodium et comprend au moins une structure lamellaire à 70 %.

5. Procédé selon les revendications 1 à 4, dans lequel la phase nettoyante comprend au moins un agent tensioactif, dans lequel ledit au moins un agent tensioactif comprend du laureth(n)-sulfate de sodium (SLEnS) où n est compris entre 1 et 3.

6. Procédé selon l'une quelconque des revendications précédentes, dans lequel le matériau contenant du zinc comprenant de la pyrithione de zinc est appliqué au moins une fois par jour pendant 14 jours ou plus.

7. Procédé selon une revendication précédente, dans lequel le matériau contenant du zinc comprenant de la pyrithione de zinc est appliqué au moins une fois par jour pendant 21 jours ou plus.

8. Procédé selon les revendications 1 à 5, dans lequel 0,5 µmg/cm² ou plus de pyrithione de zinc est déposé sur la peau non malade pendant l'application du produit de soins personnels.

9. Procédé selon l'une quelconque revendication précédente, dans lequel l'hydratation de la peau s'améliore de 0,5 unité de cornéomètre ou plus 3 heures après l'application de la composition de soins personnels à éliminer par rinçage.

10. Procédé selon l'une quelconque revendication précédente, dans lequel la peau sèche présente un grade de 2,5 ou plus avant l'application initiale de la composition de soins personnels à éliminer par rinçage.
